**Europäisches Patentamt**

**European Patent Office**

**Office européen des brevets**

(11) Veröffentlichungsnummer: **0 098 286**

**B1**

(12) # EUROPÄISCHE PATENTSCHRIFT

(45) Veröffentlichungstag der Patentschrift:
23.09.87

(51) Int. Cl.⁴: **B 03 C 1/02,** C 12 N 5/00

(21) Anmeldenummer: **83900331.6**

(22) Anmeldetag: **13.01.83**

(86) Internationale Anmeldenummer:
**PCT/DE 83/00003**

(87) Internationale Veröffentlichungsnummer:
**WO 83/02405 (21.07.83 Gazette 83/17)**

(54) **VERFAHREN UND VORRICHTUNG ZUR ABTRENNUNG VON ORGANISCHEN STOFFEN AUS EINER SUSPENSION ODER LÖSUNG.**

(30) Priorität: **14.01.82 DE 3200988**

(43) Veröffentlichungstag der Anmeldung:
**18.01.84 Patentblatt 84/3**

(45) Bekanntmachung des Hinweises auf die Patenterteilung:
**23.09.87 Patentblatt 87/39**

(84) Benannte Vertragsstaaten:
**DE FR GB SE**

(56) Entgegenhaltungen:
**EP-A-0 026 700**
**WO-A-81/00575**
**US-A-4 219 411**

**Biological Abstracts, vol. 66, May 1978, Philadelphia, Pennsylvania (US), C.S. Owen: "High gradient magnetic separation of erythrocytes", see abstract No. 25435, Biophys. J. 1978, 22 (2) 171-178 Biological Abstracts, vol. 66, May 1978, Philadelphia, Pennsylvania (US), F. Paul, et ai.: "Differential blood cell separation using a high gradient magnetic field", see abstract No. 25450, Br. J. Haemotal, 38 (2), 1978, 273-280 (Eng.)**

(73) Patentinhaber: **REED, Thomas A., Otto Hahn- Platz 7, D-6900 Heidelberg- Emmertsgrund (DE)**

(72) Erfinder: **REED, Thomas A., Otto Hahn- Platz 7, D-6900 Heidelberg- Emmertsgrund (DE)**

(74) Vertreter: **Weickmann, Heinrich, Dipl.- Ing., Patentanwälte Dipl.- Ing. H.Weickmann Dipl.- Phys.Dr. K.Fincke Dipl.- Ing. F.A.Weickmann Dipl.- Chem. B. Huber Dr.- Ing. H. Liska Dipl.- Phys.Dr. J. Prechtel Postfach 860820, D-8000 München 86 (DE)**

## Beschreibung

Die Erfindung betrifft ein Verfahren zur Abtrennung von organischen Stoffen, insbesondere Zellen, Organellen oder Proteinen, aus einer Suspension oder Lösung derselben durch selektive Adsorption an einer spezifisch wirksamen Adsorptionsschicht an der Oberfläche von (ferro) magnetischen Anlagerkörpern oder aus einer Nährstofflösung mittels einer an der Oberfläche von (ferro) magnetischen Anlagerkörpern angelagerten Zellkulturenschicht bei der Zellkulturenzucht, wobei die (ferro) magnetischen Anlagerkörper durch ein Magnetfeld in einem Reaktionsraum zurückgehalten werden.

Aus der WO-A-81/00575 ist ein derartiges Verfahren bekannt, wobei mikrobiologische Katalysatorstoffe an den ferromagnetischen Anlagerkörpern angebracht sind. Zur Rückgewinnung dieser Bio-Katalysatoren nach einer erfolgten Fermentationsreaktion werden beim Leeren des Fermentations-Reaktors die Anlagerkörper mit Hilfe magnetischer Kräfte im Reaktor zurückgehalten. Man kann statt dessen auch das zu fermentierende organische Substrat kontinuierlich durch den Reaktor fließen lassen, wobei dann ein magnetisches Feld die Anlagerkörper im Reaktor zurückhält. Über die Gestaltung des Magnetfelds sowie über die daraus resultierende räumliche Anordnung der Anlagerkörper im Reaktor gibt der Vergleich mit einer Filterscheibe in dieser Druckschrift (Seite 4, Zeile 4 sowie Zeilen 32 und 33) einen Hinweis. Die Anlagerkörper konzentrieren sich demnach im Bereich des Ausgangs des Reaktors unter Bildung eines offenen Filters ähnlich einer Trennsäule. Für eine gewünschte Abtrenn-Effektivität werden eine relativ hohe Anzahl von Anlagerkörpern benötigt.

Aus der US-A-4 219 411 ist es bekannt, ferromagnetische Anlagerkörper mit einer für organische Stoffe spezifisch wirksamen Adsorptionsschicht zu versehen und diese Anlagerkörper nach erfolgter Vermischung mit der Zellsuspension und erfolgter Anlagerung der gewünschten Stoffe mit Hilfe eines Magnetfelds von der Lösung zu trennen. Der Einfang der Anlagerkörper durch das Magnetfeld kann noch dadurch verbessert werden, daß ein ferromagnetischer Träger (Stahlwolle) in das Magnetfeld eingesetzt wird. Zur Durchführung dieses bekannten Verfahrens benötigt man folglich eine große Menge Anlagerkörper entsprechend dem Volumen der in Bearbeitung befindlichen Suspension oder Lösung.

Der Erfindung liegt die Aufgabe zugrunde, ein Verfahren der eingangs genannten Art bereitzustellen, welches mit einer geringeren Anlagerkörpermenge auskommt bei großer effektiver Anlagerkörperoberfläche. Diese Aufgabe wird dadurch gelöst, daß die Anlagerkörper im Reaktionsraum in Abstand voneinander gehalten werden, ggf. unter Verwendung eines den Reaktionsraum durchsetzenden ferromagnetischen, filigranartigen Träger, an dessen Oberfläche die Anlagerkörper aufgrund eines trägerinduzierten Magnetfeldgradienten gehalten werden, wobei das Verhältnis von Gesamtvolumen der Anlagerkörper zum Volumen des Reaktionsraums zwischen 0,01 und 0,5, vorzugsweise zwischen 0,03 und 0,2 liegt. Aufgrund der Verteilung der Anlagerkörper über den Reaktionsraum mit Abstand voneinander mit den angegebenen Volumenverhältnissen ergibt sich ein geringer Strömungswiderstand, der eine entsprechend hohe Strömungsgeschwindigkeit der Suspension (d.h. der die organischen Stoffe enthaltenden Suspension) zuläßt. Ein Steckenbleiben von organischen Stoffen zwischen benachbarten Anlagerkörpern ist praktisch ausgeschlossen. Die kurze Durchsatzzeit gestattet die Ausweitung des Verfahrens über den Laborbetrieb hinaus. Aufgrund der verkürzten Verweildauer verringert sich die Gefahr nichtspezifischer Bindung unerwünschter Zellen oder Organellen an die Anlagerkörper. Aufgrund der relativ hohen Strömungsgeschwindigkeit werden die nichtspezifischen Zellen oder Organellen in der Regel von der Strömung mitgenommen, so daß der anschließende Waschverfahrensschritt zur Entfernung dieser Stoffe an den Anlagerkörpern entfallen kann oder zumindest verkürzt werden kann. Überraschenderweise hat sich gezeigt, daß trotz der verringerten Gesamtoberfläche der Anlagerkörper im Vergleich zu einer Trennsäulenanordnung die Abtrenneffektivität bei gegebenem Reaktionsraumvolumen im wesentlichen unverändert geblieben ist und die spezifische Adsorption sogar wesentlich verbessert wurde. Dies liegt wohl daran, daß die Halbwertszeit typischer spezifischer Bindungsreaktionen zwischen den speziellen organischen Stoffen und der Adsorptionsschicht der Anlagerkörper zwischen 30 Sekunden und 20 Minuten liegt in Abhängigkeit von der Konzentration der Anlagerkörper und der Konzentration des spezifischen Substrats auf der Anlagerkörperoberfläche. Die Bindungsreaktion ist von erster Ordnung. Die Konzentration der spezifischen Zellen oder Organellen kann dabei mindestens $10^6$ pro $cm^3$ betragen. Wird demnach erfindungsgemäß die die organischen Stoffe enthaltende Suspension der Halbwertszeit entsprechend in relativ kurzer Zeit durch das Reaktionsvolumen geleitet, so erfolgen in dieser Zeit praktisch sämtliche möglichen spezifischen Bindungsreaktionen. Die Adsorptionsfläche wird demnach voll ausgenützt, während bei der bekannten Trennsäule aufgrund der die Reaktions-Halbwertszeit weit überschreitenden Verweildauer der Suspension im Reaktionsraum Folgereaktionen, insbesondere nichtspezifische Bindungsreaktionen, stattfinden, die das Abtrennergebnis trotz größerer Absorptionsfläche verschlechtern (Kontamination des Endprodukts durch nichtspezifische organische Stoffe). Von Vorteil ist auch, daß das erfindungsgemäße Verfahren mit weniger Anlagerkörpern auskommt, was wiederum die Wirtschaftlichkeit des Verfahrens verbessert. Die benötigte Menge an Anlagerkörpern hängt ausschließlich vom Volumen des durchströmten Raumes ab, auf den sich die Anlagerkörper verteilen. Es kann daher ggf. ein wesentlich größeres Suspensions- bzw. Lösungs-Volumen eingesetzt werden. Einen Verlust von Anlagerkörpern durch Mitnahme im Suspensionsstrom verhindern auf einfache Weise die Magnetfeldgradienten, die entsprechende Rückhaltekräfte auf die magnetischen, vorzugsweise ferromagnetischen, Anlagerkörper ausüben. Als Beispiele für spezifisch wirksame Adsorptionsschichten seien genannt Antikörper, Haptene oder chemische Substrate, die an der Oberfläche der Anlagerkörper kovalent gebunden sind und die wiederum ausschließlich oder bevorzugt spezielle Zellen oder Organellen binden. Um ein Anlagern der Anlagerkörper aneinander oder an Behälterwänden zu verhindern, was eine Verringerung der wirksamen Anlagerkörperoberfläche zur Folge hätte wird vorgeschlagen, daß man die Anlagerkörper mittels einer die Anlagerkörper in zur Strömungsrichtung der Suspension oder Lösung in entgegengesetzter Richtung bewegenden Rühreinrichtung über den Reaktionsraum verteilt hält.

Ferner wird vorgeschlagen, daß nach dem Hindurchleiten der Suspension durch den Reaktionsraum, vorzugsweise nach anschließendem Hindurchleiten von Waschflüssigkeit durch den Reaktionsraum, die Magnetfeldgradienten beseitigt werden und die Anlagerkörper in einem Auffangraum vorzugsweise unterhalb des Reaktionsraums zur weiteren Bearbeitung, insbesondere zum Entfernen der abgelagerten organischen Stoffe, gesammelt werden. Zum Sammeln der Anlagerkörper ist also lediglich der entsprechende Elektromagnet abzuschalten, woraufhin die Anlagerkörper in den Auffangraum fallen.

Um möglichst kontaminationsfreie Produkte zu erhalten, wird vorgeschlagen, vor dem Hindurchleiten der Suspension oder Lösung, Waschflüssigkeit durch den Reaktionsraum hindurchzuleiten.

Das erfindungsgemäße Verfahren läßt sich auch mit Erfolg zur Züchtung von Zellkulturen einsetzen, indem man eine Nährstofflösung durch den Reaktionsraum hindurchleitet, in welchem mit einer Zellkulturenschicht versehene magnetische, vorzugsweise ferromagnetische Anlagerkörper durch Magnetfeldgradienten in einer offenen Anordnung gehalten werden. Die offene Anordnung sorgt für ein ungestörtes Zellwachstum; die Anlagerkörper werden aufgrund der Magnetfeldgradienten innerhalb des Reaktionsraumvolumens gehalten, so daß nicht die Gefahr besteht, daß die Anlagerkörper von der Nährstofflösungsströmung erfaßt und weggeschwemmt werden. Man kann also Zellen in sehr hoher Konzentration züchten und dabei ständig frisches Kulturmedium an denselben vorbeifließen lassen, was eine hohe Zellwachstumsrate gewährleistet.

Die Erfindung betrifft auch eine Vorrichtung zur Durchführung des Verfahrens mit den vorstehend beschriebenen Merkmalen, umfassend einen Reaktionsbehälter mit Einlaß und Auslaß zum Durchleiten von Abtrennflüssigkeit, insbesondere Zellen, Organellen oder Proteine enthaltender Suspension oder Nährstofflösung, vorbei an einer Vielzahl von magnetischen, ggf. ferromagnetischen Anlagerkörpern innerhalb des Reaktionsbehälters und eine Magnetanordnung zur Erzeugung eines die Anlagerkörper in einem Reaktionsvolumen innerhalb des Reaktionsbehälters haltenden Magnetfeldes, wobei die Anlagerkörper mit einer für einen oder mehrere Untersuchungsstoffe spezifisch wirksamen Adsorptionsschicht oder mit einer Zellkulturenschicht versehen sind. Um mit baulich einfachen Mitteln zu erreichen, daß die Anlagerkörper ggf. mit mehr oder weniger großem Abstand voneinander im Reaktionsvolumen gehalten werden und nicht von der Strömung mitgenommen werden, wird vorgeschlagen, daß ein das Reaktionsvolumen ausfüllender, filigranartiger, ferromagnetischer Trägerkörper vorgesehen ist, an dessen Oberfläche die Anlagerkörper aufgrund durch den Trägerkörper induzierter Magnetfeldgradienten gehalten sind. Aufgrund der Filigranstruktur werden lokal Magnetfeldgradienten erzeugt, die die Anlagerkörper zur Trägerkörper-Oberfläche hin ziehen. Der Trägerkörper sorgt auf diese Weise für eine angenähert gleichmäßige Verteilung der Anlagerkörper über das Reaktionsvolumen. Da der Trägerkörper die Anlagerkörper nicht mechanisch zu fixieren hat, kann er entsprechend leicht gebaut sein mit geringem Strömungswiderstand.

Wenn, wie erfindungsgemäß vorgeschlagen, das im wesentlichen homogene Magnetfeld quer zur Strömungsrichtung verläuft, lagern sich die Anlagerkörper hauptsächlich an zur Strömungsrichtung parallelen (und zur Magnetfeldrichtung senkrechten) Flächen des Trägerkörpers an und werden daher vorteilhafterweise praktisch allseits von der Abtrennflüssigkeit umströmt.

Um die ferromagnetischen Anlagerkörper am ferromagnetischen Trägerkörper zu halten, genügt ein relativ schwaches Magnetfeld zwischen 0,005 und 0,05 Tesla, vorzugsweise 0,01 und 0,1 Tesla, am besten von etwa 0,03 Tesla.

Geringe Herstellungskosten ergeben sich dann, wenn der Trägerkörper aus Draht, vorzugsweise Edelstahldraht, gebildet ist mit einem im Bereich der Abmessung der Anlagerkörper liegenden Drahtdurchmesser. Bei einem derartigen Drahtdurchmesser sind die auf den Anlagerkörper wirkenden Magnetkräfte am größten.

Der Drahtkörper kann gitter- oder siebartig aufgebaut sein oder aus willkürlich gebogenem Draht (ähnlich Stahlwolle) gebildet sein.

Eine ausreichende Konzentration der Anlagerkörper bei vorteilhaft geringem Strömungswiderstand ist gewährleistet, wenn der mittlere Abstand zwischen benachbarten Drahtstücken das 5- bis 200-fache, vorzugsweise das 10- bis 100-fache, des Drahtdurchmessers beträgt.

Das gewünschte, quer zur Strömungsrichtung verlaufende homogene Magnetfeld erhält man in einfacher Weise dadurch, daß der Reaktionsbehälter zwischen die Polschuhe eines Magneten eingesetzt ist.

Anstelle der magnetischen Fixierung der Anlagerkörper am Trägerkörper kann vorgesehen sein, daß von der Magnetanordnung zumindest am Ausströmende des Reaktionsvolumens, an welchem die Abtrennflüssigkeit das Reaktionsvolumen verläßt, ein im Falle ferromagnetischer Anlagerkörper nach außen hin abfallender Magnetfeldgradient erzeugt wird, und daß eine Rühreinrichtung für die Anlagerkörper vorgesehen ist, welche für eine Verteilung der Anlagerkörper über das Reaktionsvolumen sorgt. Dieser Magnetfeldgradient übt eine rücktreibende Kraft auf die von der Strömung der Suspension bzw. Lösung mitgenommenen Anlagerkörper aus und hält diese im Reaktionsvolumen zurück. Der genannte Trägerkörper kann entfallen, so daß die Anlagerkörper im Reaktionsvolumen frei beweglich sind und deren gesamte Oberfläche zur spezifischen Anlagerung zur Verfügung steht. Die Rühreinrichtung verhindert, daß die Anlagerkörper sich im Reaktionsvolumen zusammenballen oder sich an den Behälterwänden ablagern.

Man erhält mit baulich einfachen Mitteln den gewünschten Magentfeldgradienten dadurch, daß der vorzugsweise zylinderförmige Reaktionsbehälter in die Bohrung einer Zylinderspule eingesetzt ist.

Die Rühreinrichtung wird vorzugsweise von einem rotierenden, ggf. zur Reaktionsbehälterachse konzentrischen Rührstab gebildet, der die Anlagerkörper in zur Strömungsrichtung der Abtrennflüssigkeit entgegengesetzter Richtung bewegt. Auf diese Weise ist mit baulich einfachen Mitteln sichergestellt, daß sich die Anlagerkörper während es Betriebs gleichmäßig über das gesamte Reaktionsvolumen verteilen.

Um die Zufuhr der Anlagerkörper in den Reaktionsraum mit einfachen Mitteln bewerkstelligen zu können, wird vorgeschlagen, den Reaktionsbehälter mit einer Einführöffnung für eine Hohlnadel oder einen Schlauch zur Zuführung von Anlagerkörpern zu versehen. Die Einführöffnung kann daneben auch als Entlüftungsöffnung dienen.

Zur Sammlung der Anlagerkörper nach der erfolgten Reaktion wird vorgeschlagen, einen vorzugsweise mit einem Ablaßventil versehenen Auffangraum für die Anlagerkörper unterhalb des Reaktionsvolumens vorzusehen.

Eine vorteilhaft große wirksame Oberfläche bei gegebenem Volumenanteil der Anlagerkörper erhält man dann, wenn die vorzugsweise im wesentlichen kugelförmigen Anlagerkörper Abmessungen zwischen 10 und 200 μm, vorzugsweise zwischen 25 und 100 μm aufweisen.

Die Erfindung wird im folgenden an Hand der Zeichnung an zwei Ausführungsbeispielen erläutert.

Es zeigt:

Fig. 1 eine Seitenansicht einer erfindungsgemäßen Abtrennvorrichtung für organische Stoffe;

Fig. 2 das Kraftfeld um einen ferromagnetischen Draht in einem homogenen Magnetfeld;

Fig. 3 eine Seitenansicht einer zweiten Ausführungsform der erfindungsgemäßen Abtrennvorrichtung;

Fig. 4 eine Prinzipskizze des Magnetfeldverlaufs in der Spule der Vorrichtung gemäß Fig. 3.

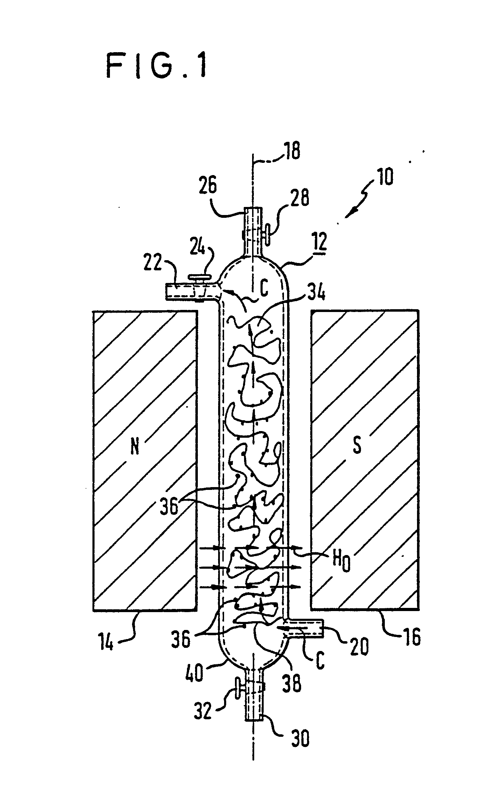

Die in Fig. 1 dargestellte Abtrennvorrichtung 10 besteht aus einem Reaktionsbehälter 12, der zwischen die Polschuhe 14 und 16 eines nicht näher dargestellten Elektromagneten eingesetzt ist. Der in Fig. 1 linke Polschuh 14 ist beispielsweise der Nordpol, der Polschuh 16 ist demgemäß der Südpol. Zwischen beiden Polschuhen herrscht ein im wesentlichen homogenes Magnetfeld $H_0$.

Der Reaktionsbehälter 12 ist im wesentlichen zylindrisch mit zur Magnetfeldrichtung $H_0$ senkrechter, vertikaler Zylinderachse 18. An seinem unteren Ende steht in Fig. 1 nach rechts ab ein Schlauchanschlußstutzen 20, dem durch eine nicht dargestellte Zuleitung Zellen oder Organellen enthaltende Suspension zugeführt wird. Über einen am oberen Ende des Reaktionsbehälters 12 nach links abstehenden weiteren Schlauchanschlußstutzen 22 wird die Suspension wieder abgeleitet. Im oberen Schlauchanschlußstutzen 22 ist ein Absperrventil 24 in den aus Glas bestehenden Reaktionsbehälter 12 eingeschliffen.

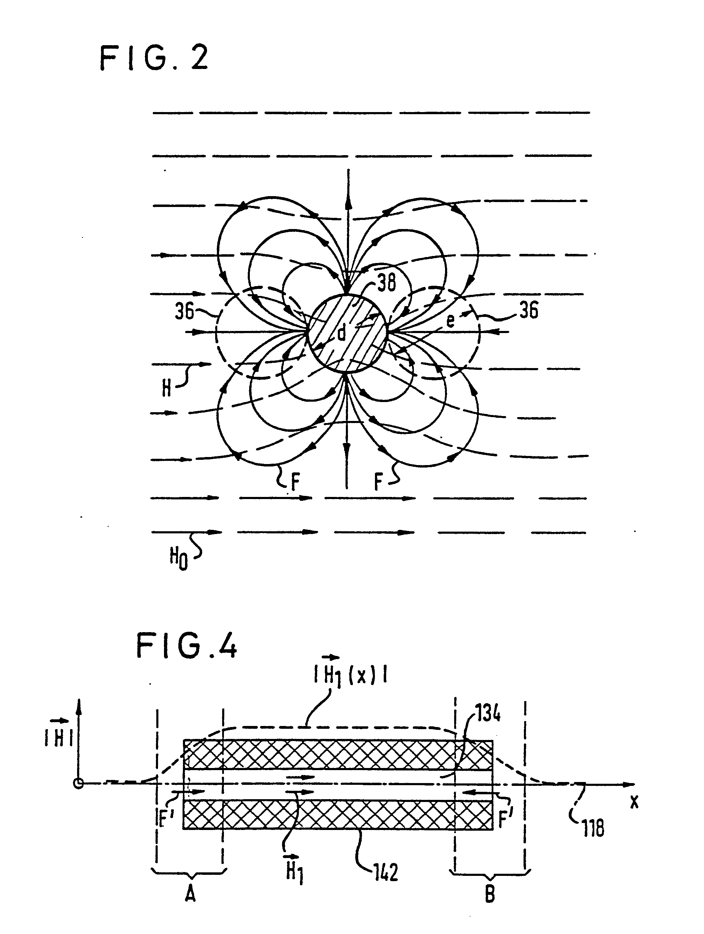

Am oberen Ende des Reaktionsbehälters 12 steht ein Einführstutzen 26 ab, durch den im folgenden mit Perlen bezeichnete Anlagerkörper in den Reaktionsbehälterinnenraum eingeführt werden können. Der Einführstutzen 26 ist mittels eines Ventils 28 absperrbar. Dementsprechend ist am unteren Ende des Reaktionsbehälters 12 ein Abgabestutzen 30 angeformt, der ebenfalls durch ein Ventil 32 absperrbar ist. In einem mit Reaktionsvolumen 34 bezeichneten Teil des Reaktionsbehälterinnenraums im Bereich zwischen den Polschuhen 14 und 16 ist ein als Träger für die Perlen 36 dienender, willkürlich gebogener äußerst feiner Stahldraht 38 eingesetzt; es können auch mehrere derartige Stahldrähte in das Reaktionsbehälterinnere eingeführt sein. Der Stahldraht hat einen Durchmesser d (siehe Fig. 2) von 25 bis 100 μm; der Stahldraht 38 ist derart locker nach Art von Stahlwolle zusammengeballt, daß benachbarte Drahtstücke einen durchschnittlichen Abstand voneinander entsprechend dem 10- bis 100-fachen Durchmesser d haben. Die Stärke des Magnetfeldes $H_0$ beträgt etwa 0,03 Tesla (300 Gauss).

Die Perlen 36 bestehen aus kleinen Kugeln aus Adsorptionsmaterial wie Polyagarose oder Polyacrylamid und enthalten Einschlüsse aus ferromagnetischem Material. Im Handel sind mehrere Typen dieser Perlen erhältlich. Zur spezifischen Bindung bestimmter Zellen, Organellen oder Protein moleküle wird auf die Oberfläche der Perlen 36 ein Substrat aufgetragen, welches an die Perlenoberfläche kovalent gebunden ist; es kann jedoch auch ein ebenfalls kovalent gebundener kurzkettiger, sogenannter "Spacer" verwendet werden. Der Durchmesser e der Perlen 36 (Fig. 2) beträgt etwa 100 μm.

Aufgrund des Magnetfeldes $H_0$ werden die Perlen 36 von den Windungen des Stahldrahts 38 magnetisch angezogen und an der Drahtoberfläche festgehalten, so daß die Perlen 36 trotz der Strömung der Suspension durch den Reaktionsbehälter 12 in Richtung der Pfeile C im Reaktionsvolumen 34 bleiben. In Fig. 2 sind mit durchgezogener Linie die auf einen ferromagnetischen Körper einwirkenden Kraftlinien F dargestellt. Diese kommen aufgrund der Konzentrierung der mit unterbrochenen Linien angedeuteten Magnetfeldlinien H im Bereich des ferromagnetischen Drahts 38 zustande. Wie beispielsweise in der Zeitschrift IEEE Transactions on Magnetics, VOL.MAG-9, NO. 3, September 1973, Seiten 303-306 ausgeführt ist, ist diese vom Magnetfeld $H_0$ sowie vom lokalen Magnetfeldgradienten abhängige Kraft am größten, wenn der Durchmesser d des Drahts 38 etwa dem Durchmesser e des angezogenen Teilchens, hier der ferromagnetischen Perle 36, entspricht. Wie Fig. 2 zu entnehmen ist, lagern sich bei der dargestellten geometrischen Anordnung die Perlen 36 in Richtung des Felds $H_0$ an der einen oder anderen Seite (in Fig. 2 rechts oder links) des Drahts 38 an, wohingegen in zur Feldrichtung $H_0$ senkrechter Ebene eine Abstoßung der Perlen 36 erfolgt.

In Fig. 1 sind in nicht maßstabsgerechter Darstellung einige Windungen des Drahts 38 sowie einige am Draht magnetisch festgehaltene Perlen 36 gezeigt. Tatsächlich beträgt das Gesamtvolumen der Perlen 36 etwa 20 % des Volumens des Reaktionsraums 34 zwischen den Polschuhen 14 und 16.

Das erfindungsgemäße Verfahren zur Abtrennung bestimmter biologischer Zellen oder Organellen (gegebenenfalls auch Proteine) aus einer organischen Suspension geht wie folgt vonstatten.

Als erstes werden die Perlen bei angeschaltetem Elektromagneten über den Einführstutzen 26 beispielsweise mittels einer Kanüle in das Reaktionsvolumen 34 eingeführt, wobei dieses zugleich mit Pufferlösung aufgefüllt wird. Aufgrund des Magnetfelds $H_0$ und der resultierenden Magnetfeldgradienten im Oberflächenbereich des Drahts 38 werden die Perlen 36 an die Drahtoberfläche angezogen und dort festgehalten. Als nächstes wird Waschflüssigkeit über die Stutzen 20 und 22 durch den Reaktionsbehälter 12 geleitet, um gegebenenfalls vorhandenen Schmutz oder sonstige unerwünschten Stoffe aus dem Reaktionsbehälter 12 zu entfernen. Nun mehr wird die die abzutrennenden Zellen oder Organellen enthaltende Suspension durch den Reaktionsbehälter 12 geleitet, gegebenenfalls mehrfach im Kreislauf. Der Strömungswiderstand der Füllung des Reaktionsbehälters 12 aus Draht 38 und Perlen 36 ist vergleichsweise gering, so daß eine relativ hohe Strömungsgeschwindigkeit der Suspension ohne Schwierigkeiten erreicht wird. Die insgesamte Verweildauer der Suspension innerhalb des Reaktionsbehälters 12 beträgt das bis zu Vierfache der Reaktionshalbwertszeit der spezifischen Bindungsreaktion der abzutrennenden Zellen oder Organellen.

Anschließend wird Waschflüssigkeit durch den Reaktionsbehälter 12 geleitet, um an den Perlen 36 nichtspezifisch gebundene organische Substanzen abzuwaschen.

Schließlich wird der Elektromagnet abgeschaltet, so daß das Magnetfeld $H_0$ sowie die entsprechenden Magnetfeldgradienten im Bereich des Drahts 38 verschwinden. Die Perlen 36 fallen daraufhin nach unten und sammeln sich am unteren, angenähert trichterförmigen Ende 40 des Reaktionsbehälters 12. Nur mehr können durch Öffnen des Ventils 32 die Perlen 36 aus dem Behälter 12 entfernt werden und in einem weiteren Verfahrensschritt derart behandelt werden, daß sich die spezifisch gebundenen Zellen und Organellen (oder Proteine) von den Perlen 36 lösen.

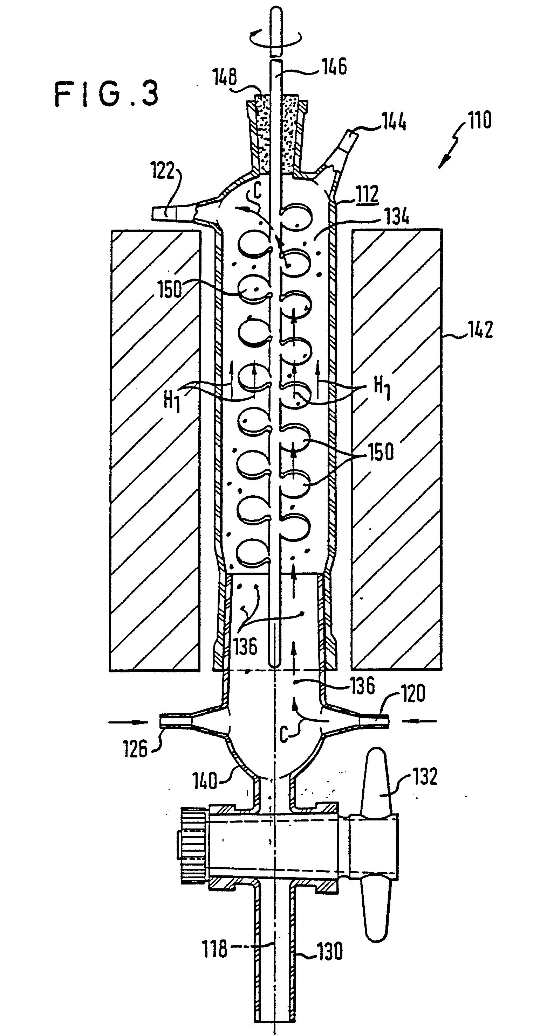

In den Fig. 3 und 4 ist eine weitere, mit 110 bezeichnete Ausführungsform der erfindungsgemäßen Abtrennvorrichtung dargestellt. Diese unterscheidet sich von der Abtrennvorrichtung gemäß Fig. 1 und 2 im wesentlichen darin, daß die ferromagnetischen Perlen 136 im mittleren Bereich des Reaktionsbehälters 112 frei beweglich sind und durch magnetische Feldgradienten daran gehindert werden, das Reaktionsvolumen 134 zu verlassen, welches wiederum im wesentlichen von dem Teil des Reaktionsbehälterinnenraums gebildet wird, welches innerhalb des Elektromagneten liegt. Der Magnet wird nunmehr von einer Zylinderspule 142 gebildet.

Der Reaktionsbehälter 112 ist wiederum mit einem unteren Schlauchanschlußstutzen 120 und einem oberen Schlauchanschlußstutzen 122 versehen, durch die die organische Suspension zu- bzw. abgeführt wird. Am unteren Behälterende ist in gleicher Weise ein Abgabestutzen 130 vorgesehen, der mittels eines Ventils 132 absperrbar ist. Am unteren Behälterende ist ferner noch ein Einführstutzen 126 zur Zuführung der Perlen 136 ausgeformt. Dieser Einführstutzen kann jedoch entfallen, wenn eine am oberen Reaktionsbehälterende vorgesehene Entlüftungsöffnung 144 daneben noch als Einführöffnung für eine Perlenzuführnadel oder einen Perlenzuführschlauch verwendet wird.

Am oberen Reaktionsbehälterende ist ein den Behälterinnenraum durchsetzender Rührstab 146 mittels eines vereinfacht dargestellten Drehlagers 148 drehbar gelagert. Der Rührstab 146 ist zur Zylinderachse 118 des im wesentlichen zylinderförmigen Reaktionsbehälters 112 zentrisch angeordnet. Er trägt im Bereich des Reaktionsvolumens 134 propellerähnlich verdrehte Flügel 150 mit etwa kreisförmigem Umriß, die bei einer entsprechenden Drehung des Stabs 146 dafür sorgen, daß die an die Flügel 150 stoßenden Perlen 136 nach unten gestoßen werden und damit entgegen der Strömungsrichtung (Pfeile C) der Suspension. Der aus Glas bestehende Reaktionsbehälter 112 ist zweiteilig, um das Einsetzen des Rührstabs 146 zu ermöglichen. Dementsprechend kann auch der Reaktionsbehälter 12 gemäß Fig. 1 zweiteilig ausgebildet sein.

Die Magnetfeldgradienten am oberen und unteren Ende der Spule 142 sorgen dafür, daß die ferromagnetischen Perlen 136 das Reaktionsvolumen 134, d.h. den Raum innerhalb der Spule, nicht verlassen. Dies soll anhand von Fig. 4 erläutert werden. Zusätzlich zum Querschnitt der um 90° gedrehten Zylinderspule 142 ist mit unterbrochener Linie der Betrag des Magnetfelds $H_1$ am Ort der Zylinderachse 118 eingetragen; die nach rechts abgetragene Ortskoordinate x gilt sowohl für die Spule 142 als auch für die Darstellung des Betrags des Magnetfelds $H_1$; der Betrag des Magnetfelds $H_1$ ist nach oben abgetragen.

Man erkennt, daß das Magnetfeld $H_1$ im zentralen Bereich des Spuleninneren einen konstanten Wert annimmt, im Bereich beider Enden abfällt und schließlich in größerer Entfernung von der Spule 142 Null wird. Die Übergangsbereiche an beiden Enden mit sich stetig änderndem Magnetfeldbetrag sind mit A bzw. B bezeichnet. In den Bereichen A und B herrscht demnach ein Magnetfeldgradient, der auf ferromagnetische (oder auch paramagnetische) Teilchen eine Kraft in Richtung zum größeren Magnetfeld hin, also zum Spuleninneren, ausübt. Die entsprechenden, mit F' bezeichneten Kraftpfeile sind in Fig. 4 angedeutet. Da das Magnetfeld in radialer Richtung im Bohrungsbereich der Spule näherungsweise konstant ist, wirken auch auf außerhalb der Achse 118 liegende ferromagnetische Teilchen in den Bereichen A und B magnetische Kräfte, die ins Spuleninnere gerichtet sind. Im Spuleninneren angeordnete ferromagnetische Teilchen werden also durch die Gradienten an beiden Spulenenden an einem Entweichen gehindert. Innerhalb des zentralen Spulenbereichs zwischen den Endabschnitten A und B gemäß Fig. 4 sind die ferromagnetischen Teilchen dagegen frei beweglich, da aufgrund des hier konstanten Magnetfelds keinerlei Magnetfeldgradienten herrschen. Anstelle des beschriebenen focussierenden Magnetfeldes kommt bei anderen Magnetformen auch prinzipiell ein Magnetfeld mit einfachem Feldgradienten in Frage.

Die Abtrennvorrichtung 110 wird im wesentlichen in gleicher Weise zum Abtrennen von organischen Stoffen aus Suspensionen eingesetzt wie die Abtrennvorrichtung 10 gemäß Fig. 1 und 2. Nach dem Zuführen der ferromagnetischen Perlen 136 beispielsweise durch die Öffnung 144 mittels einer entsprechenden Hohlnadel bei eingeschalteter Magnetspule 142 und flüssigkeitsgefülltem Behälter 112 werden die Perlen 136 durch die Magnetfeldgradienten am unteren und oberen Spulenende daran gehindert, einerseits schwerkraftbedingt in das untere Behälterende 140 außerhalb der Spule 142 zu fallen und andererseits von der Strömung der Suspension (in Richtung der Pfeile C) mitgenommen zu werden, um schließlich den Behälter 112 durch das Schlauchanschlußstück 122 zu verlassen. Nach Hindurchleiten von Waschflüssigkeit durch den Behälter 112 wird die Suspension durch den Behälter 112 geleitet. Der rotierende Rührstab 146 (Drehgeschwindigkeit beispielsweise 30 Umdrehungen/min) sorgt zum einen dafür, daß die Perlen 136 weder zusammenklumpen noch sich an der Behälterinnenwand anlagern, und zum anderen dafür, daß bei höherer Strömungsgeschwindigkeit der Suspension der Tendenz der Perlen 136, sich am oberen Spulenende zu sammeln, entgegengewirkt wird.

Nach erfolgter Anlagerung der spezifischen Zellen, Organellen oder Proteine an die Perlen wird die Suspensionszufuhr unterbrochen und gegebenenfalls Waschflüssigkeit durch den Behälter 112 geleitet. Anschließend wird der Magnet 142 abgeschaltet, so daß die Perlen 136 in das untere Ende 140 des Behälters 112 fallen und durch das Ventil 132 zur weiteren Behandlung abgelassen werden können.

Die vorbeschriebenen Abtrennvorrichtungen 10 und 110 dienen allgemein dazu, in einem durchströmten Reaktionsvolumen als Anlagerkörper dienende Perlen mit Abstand voneinander, also in einer offenen Anordnung, zu halten. Die Perlen können hierbei innerhalb des Reaktionsvolumens in einer statischen Anordnung im wesentlichen ortsunveränderlich gehalten sein (Fig. 1 und 2) oder in einer dynamischen Anordnung innerhalb des Volumens frei beweglich sein (Fig. 3 und 4). Als durch das Reaktionsvolumen zu leitendes Medium kommt, wie vorstehend beschrieben, eine Suspension aus Zellen, Organellen oder Proteinen in Frage; die Perlen dienen hierbei als Adsorptionsperlen, an denen sich die aus der Suspension abzutrennenden Zellen oder Organellen anlagern.

Die statische oder dynamische offene Anordnung von Perlen kann jedoch auch zur Zellkulturenzüchtung mit Erfolg verwendet werden. Hierzu werden in einem ersten Schritt die Anlagerkörper mit Zellkulturen "geimpft", woraufhin sich eine mehr oder weniger große Zellkulturenschicht auf den Perlen bildet. Dieses Impfen kann innerhalb des Reaktionsraumvolumens, also innerhalb der Vorrichtung 10 bzw. 110, vorgenommen werden, zum eigentlichen Zellkulturenzüchten wird anschließend Nährstofflösung durch das Reaktionsraumvolumen hindurchgeleitet, wobei die Zellkulturen-Perlen durch die Magnetfeldgradienten in der offenen statischen oder dynamischen Anordnung gehalten werden. Magnetfeldgradienten sorgen also dafür, daß die ferromagnetischen Perlen nicht von der Nährstofflösungsströmung mitgenommen werden. Da die Perlen nur einen relativ geringen Teil des Reaktionsraumvolumens ausfüllen, können große Mengen an Zellkulturen gezüchtet werden. Da ständig Nährstofflösung an den Zellen vorbeigeführt wird, ergibt sich eine hohe Zellkulturenerzeugungsgeschwindigkeit.

## Patentansprüche

1. Verfahren zur Abtrennung von organischen Stoffen, insbesondere Zellen, Organellen oder Proteinen, aus einer Suspension oder Lösung derselben durch selektive Adsorption an einer spezifisch wirksamen Adsorptionsschicht an der Oberfläche von (ferro) magnetischen Anlagerkörpern oder aus einer Nährstofflösung mittels einer an der Oberfläche von (ferro) magnetischen Anlagerkörpern angelagerten Zellkulturenschicht bei der Zellkulturenzucht, wobei die (ferro) magnetischen Anlagerkörper durch ein Magnetfeld in einem durchströmten Reaktionsraum zurückgehalten werden,
dadurch gekennzeichnet,
daß die Anlagerkörper im Reaktionsraum in Abstand voneinander gehalten werden, ggf. unter Verwendung eines den Reaktionsraum durchsetzenden ferromagnetischen, filigranartigen Trägers, an dessen Oberfläche die Anlagerkörper aufgrund eines trägerinduzierten Magnetfeldgradienten gehalten werden, wobei das Verhältnis von Gesamtvolumen der Anlagerkörper zum Volumen des Reaktionsraums zwischen 0,01 und 0,5, vorzugsweise zwischen 0,03 und 0,2 liegt.

2. Verfahren nach Anspruch 1,
dadurch gekennzeichnet,
daß man die Anlagerkörper mittels einer die Anlagerkörper in zur Strömungsrichtung der Suspension oder Lösung in entgegengesetzter Richtung bewegenden Rühreinrichtung über den Reaktionsraum verteilt hält.

3. Verfahren nach Anspruch 1 oder 2,
dadurch gekennzeichnet,
daß nach dem Hindurchleiten der Supension oder Lösung durch den Reaktionsraum, vorzugsweise nach anschließendem Hindurchleiten von Waschflüssigkeit durch den Reaktionsraum, die Magnetfeldgradienten beseitigt werden und die Anlagerkörper in einem Auffangraum vorzugsweise unterhalb des Reaktionsraums zur weiteren Bearbeitung, insbesondere zum Entfernen der abgelagerten organischen Stoffe, gesammelt werden.

4. Verfahren nach einem der vorhergehenden Ansprüche,
dadurch gekennzeichnet,
daß, vor dem Hindurchleiten der Suspension oder Lösung, Waschflüssigkeit durch den Reaktionsraum hindurchgeleitet wird.

5. Vorrichtung zur Durchführung des Verfahrens nach einem der vorhergehenden Ansprüche, umfassend einen Reaktionsbehälter (10;) mit Einlaß (20;) und Auslaß (22;) zum Durchleiten von Abtrennflüssigkeit, insbesondere Zellen, Organellen oder Proteine enthaltender Suspension oder Nährstofflösung, vorbei an einer Vielzahl von magnetischen, ggf. ferromagnetischen Anlagerkörpern (36;) innerhalb des Reaktionsbehälters (12;) und eine Magnetanordnung (14, 16, 38;) zur Erzeugung eines die Anlagerkörper (36;) in einem Reaktionsvolumen (34;) innerhalb des Reaktionsbehälters (12;) haltenden Magnetfeldes, wobei die Anlagerkörper mit einer für einen oder mehrere Untersuchungsstoffe spezifisch wirksamen Adsorptionsschicht oder mit einer Zellkulturenschicht versehen sind,
dadurch gekennzeichnet,
daß ein das Reaktionsvolumen (34) ausfüllender, filigranartiger, ferromagnetischer Trägerkörper (38) vorgesehen ist, an dessen Oberfläche die Anlagerkörper (36) aufgrund durch den Trägerkörper (38) induzierter Magnetfeldgradienten gehalten sind.

6. Vorrichtung nach Anspruch 5,
dadurch gekennzeichnet,
daß das von der Magnetanordnung (14, 16, 38) erzeugte Magnetfeld ($H_0$) quer zur Strömungsrichtung der Suspension bzw. Nährstofflösung verläuft.

7. Vorrichtung nach Anspruch 5 oder 6,
dadurch gekennzeichnet,
daß die Stärke des Magnetfeldes ($H_0$) zwischen 0,005 und 0,05 Tesla, vorzugsweise zwischen 0,01 und 0,1 Tesla, am besten etwa bei 0,03 Tesla, liegt.

8. Vorrichtung nach einem der Ansprüche 5 bis 7,
dadurch gekennzeichnet,
daß der Trägerkörper aus Draht (38), vorzugsweise Edelstahldraht, gebildet ist mit einem im Bereich der Abmessungen (Durchmesser e) der Anlagerkörper (36) liegenden Drahtdurchmesser (d).

9. Vorrichtung nach Anspruch 8,
dadurch gekennzeichnet,
daß der Trägerkörper gitter- oder siebartig aufgebaut ist.

10. Vorrichtung nach Anspruch 9,
dadurch gekennzeichnet,
daß der Trägerkörper aus willkürlich gebogenem Draht (38) gebildet ist.

11. Vorrichtung nach einem der Ansprüche 8 bis 10,
dadurch <u>gekennzeichnet</u>
daß der mittlere Abstand zwischen benachbarten Drahtstücken das 5- bis 200-fache, vorzugsweise das 10- bis 100-fache, des Drahtdurchmessers (d) beträgt.

12. Vorrichtung nach einem der Ansprüche 4 bis 10,
dadurch <u>gekennzeichnet</u>,
daß der Reaktionsbehälter (12) zwischen die Polschuhe (14, 16) eines Magneten eingesetzt ist.

13. Vorrichtung zur Durchführung des Verfahrens nach einem der Ansprüche 1 bis 4, umfassend einen Reaktionsbehälter (110) mit Einlaß (120) und Auslaß (122) zum Durchleiten von Abtrennflüssigkeit, insbesondere Zellen, Organellen oder Proteine enthaltender Suspension oder Nährstofflösung, vorbei an einer Vielzahl von magnetischen, ggf. ferromagnetischen Anlagerkörpern (136) innerhalb des Reaktionsbehälters (112) und eine Magnetanordnung (142) zur Erzeugung eines die Anlagerkörper (136) in einem Reaktionsvolumen (134) innerhalb des Reaktionsbehälters (112) haltenden Magnetfeldes, wobei die Anlagerkörper mit einer für einen oder mehrere Untersuchungsstoffe spezifisch wirksamen Adsorptionsschicht oder mit einer Zellkulturenschicht versehen sind,
dadurch <u>gekennzeichnet</u>,
daß von der Magnetanordnung zumindest am Ausströmende des Reaktionsvolumens (134), an welchem die Abtrennflüssigkeit das Reaktionsvolumen (134) verläßt, ein im Falle ferromagnetischer Anlagerkörper (136) nach außen hin abfallender Magnetfeldgradient erzeugt wird, und daß eine Rühreinrichtung für die Anlagerkörper (136) vorgesehen ist, welche für eine Verteilung der Anlagerkörper über das Reaktionsvolumen (134) sorgt.

14. Vorrichtung nach Anspruch 13,
dadurch <u>gekennzeichnet</u>,
daß der vorzugsweise zylinderförmige Reaktionsbehälter (112) in die Bohrung einer Zylinderspule (142) eingesetzt ist.

15. Vorrichtung nach Anspruch 13 oder 14,
<u>gekennzeichnet</u> durch
einen rotierenden, ggf. zur Reaktionsbehälterachse (118) konzentrischen Rührstab (146), der die Anlagerkörper (136) in zur Strömungsrichtung (C) der Abtrennflüssigkeit entgegengesetzter Richtung bewegt.

16. Vorrichtung nach einem der Ansprüche 5 bis 15,
<u>gekennzeichnet</u> durch
eine vorzugsweise zur Entlüftung dienende Einführöffnung (26; 144) des Reaktionsbehälters (12; 112) für eine Hohlnadel oder einen Schlauch zur Zuführung von Anlagerkörpern (36; 136) in das Reaktionsvolumen (34; 134).

17. Vorrichtung nach einem der Ansprüche 5 bis 16,
<u>gekennzeichnet</u> durch

einen vorzugsweise mit einem Ablaßventil (32; 132) versehenen Auffangraum (40; 140) für die Anlagerkörper (36; 136) unterhalb des Reaktionsvolumens (34; 134).

18. Vorrichtung nach einem der Ansprüche 5 bis 17,
dadurch <u>gekennzeichnet</u>,
daß die vorzugsweise im wesentlichen kugelförmigen Anlagerkörper (36; 136) Abmessungen (Durchmesser e) zwischen 10 und 200 μm, vorzugsweise zwischen 25 und 100 μm, aufweisen.

## Claims

1. Method for separating organic substances, especially cells, organellae or proteins, out of a suspension or solution thereof by selective adsorption on an adsorption layer of specific effect on the surface of (ferro-) magnetic fixing bodies or out of a nutrient solution by means of a cell culture layer fixed on the surface of (ferro-) magnetic fixing bodies in cell culture cultivation, where the (ferro-) magnetic fixing bodies are held back by a magnetic field in a reaction space through which flow takes place, characterised in that the fixing bodies are held spaced from one another in the reaction space, possibly, by the use of a ferro-magnetic carrier of filigree type penetrating the reaction space, on the surface of which the fixing bodies are hold by reason of a carrier-induced magnetic field gradient, the ratio of the total volume of the fixing bodies to the volume of the reaction space lying between 0.01 and 0.5, preferably between 0.03 and 0.2.

2. Method according to Claim 1, characterised in that the fixing bodies are held in distribution over the reaction space by means of an agitator which moves the fixing bodies in a direction opposite to the direction of flow of the suspension or solution.

3. Method according to Claim 1 or 2, characterised in that after the conducting of the suspension or solution through the reaction space, preferably after subsequent conducting of washing liquid through the reaction space, the magnetic field gradients are eliminated and the fixing bodies are collected in a collection space preferably below the reaction space for further processing, especially for the removal of the deposited organic substances.

4. Method according to one of the preceding Claims, characterised in that before the suspension or solution is conducted through, washing liquid is conducted through the reaction space.

5. Apparatus for carrying out the method according to one of the preceding Claims, comprising a reaction vessel (10) having an inlet (20) and an outlet (22) for the conducting through of separation liquid, especially suspension containing cells, organellae or proteins, or nutrient solution, past a plurality of magnetic, possibly ferro-magnetic, fixing bodies (36) within the reaction vessel (12) and a magnet arrangement (14, 16, 38) for producing as magnetic field holding the fixing bodies (36) in a reaction volume (34) within the reaction vessel (12), the fixing bodies being provided whith an adsorption layer specifically effective for one or more examination substances or with a cell culture layer, characterised in that a filigree-type, ferro-magnetic carrier body (38) filling out the reaction volume (34) is provided on the suface of which the fixing bodies (36) are held by reason of magnetic field gadients induced by the carrier body (38).

6. Apparatus according to Claim 5, characterised in that the magnetic field ($H_0$) produced by the magnet arrangement (14, 16, 38) extends transversely of the direction of flow of the suspension or nutrient solution.

7. Apparatus according to Claim 5 or 6, characterised in that the power of the magetic field ($H_0$) lies between 0.005 and 0.05 Tesla, preferably between 0.01 and 0.1 Tesla, optimallt at about 0.03 Tesla.

8. Apparatus according to one of Claims 5 to 7, characterised in that the carrier body is formed from wire (38), preferably high-grade steel wire, with a wire diameter (d) lying in the region of the dimensions (diameter e) of the fixing bodies (36).

9. Apparatus according to Claim 8, characterised in that the carrier body is of lattice-type or sieve-type formation.

10. Apparatus according to Claim 9, characterised in that the carrier body is formed from deliberataly bent wire (38).

11. Apparatus according to one of Claims 8 to 10, characterised in that the mean distance between adjacent wire pieces amounts to 5 to 200 times, preferably 10 to100 times, the whire diameter (d).

12. Apparatus according to one of Claims 4 to 10, characterised in that the reaction vessel (12) is inserted between the pole shoes (14, 16) of a magnet.

13. Apparatus for carrying out the method according to one of Claims 1 to 4, comprising a reaction vessel (110) with inlet (120) and outlet (122) for the conducting through of separation liquid, especially suspension containing cells, organellae or proteins or nutrient solution, past a plurality of magnetic, possibly ferro-magnetic, fixing bodies (136) within the reaction vessel (112) and a magnet arrangement (142) for the generation of a magnetic field holding the fixing bodies (136) in a reaction volume (134) within the reaction vessel.(112), the fixing bodies being provided with an adsorption layer specifically effective for one or more examination substances or with a cell culture layer, characterised in that the magnet arrangement generates, at least at the outflow end of the reaction volume (134) where the separation liquid departs from the reaction volume (134), a magnetic field gradient falling off towards the exterior in the case of ferro-magetic fixing bodies (136), and in that an agitator for the fixing bodies (136) is provided which ensures a distribution of the fixing bodies over the reaction volume (134).

14. Apparatus according to Claim 13, characterised in that the preferably cylindrical reaction vessel (112) is inserted into the bore of a cylindrical coil (142).

15. Apparatus according to Claim 13 or 14, characterised by a rotating stirring rod (146) possibly concentric with the axis of the reaction vessel (118), which rod moves the fixing bodies (136) in a direction opposite to the direction (C) of flow of the separation

16. Apparatus according to one of Claims 5 to 15, characterised by an insertion opening (26; 144) of the reaction vessel (12; 112), preferably serving for air outlet, for a hollow needle or a hose for the feed of fixing bodies (36; 136) into the reaction volume (34; 134).

17. Apparatus according to one of Claims 5 to 16, characterised by a collecting space (40; 140), preferably provided with an outlet valve (32; 132), for the fixing bodies (36; 136), beneath the reaction volume, (34, 134).

18. Apparatus according to one of Claims 5 to 17, characterised in that the preferably substantielly spherical fixing bodies (36; 136) possess dimensions (diemeter e) beetween 10 and 200 µm, preferebly between 25 and 100 µm.

## Revendications

1. Procédé de séparation de substances organiques et plus specialement de cellules-organelles ou protéines provenant d'une suspension ou d'une solution par adsorption sélective sur une couche d'adsorption sélectivement efficace sur la surface du corps de fixation (ferro)magnétiques ou provenant d'une solution nutritive au moyen d'une couche de cultures de cellules déposée sur la surface de corps de fixation (ferro)magnétiques lors de la culture de cellules, les corps de fixation (ferro)magnétiques étant maintenus par un champ magnétique dans un compartiment de réaction traversé par un écoulement, caractérisé en ce que les corps de fixation sont maintenus à une certaine distance les une des autres dans le compartiment de réaction, éventuellement par utilisation d'un support ferromagnétique de type filigrane occupant la chambre de réaction, à la surface duquel sont maintenus les corps de fixation par des gradients de champ magnétique induit par le support, le rapport entre le volume total des corps de fixation et le volume de la chambre de reaction se situant entre 0,01 et 0,5 et de préférence entre 0,03 et 0,2.

2. Procédé selon la revendication 1, caractérisé en ce que l'on maintient les corps de fixation à l'état dispersé dans la chambre de réaction au moyen d'un dispositif d'agitation déplaçant les corps de fixation dans une direction contraire à la direction de l'écoulement de la suspension ou de la solution.

3. Procédé selon la revendication 1 ou la revendication 2, caractérisé en ce que, après que la suspension ou la solution ait traversé la chambre de réaction, de préférence une fois que le liquide de lavage ait fini de traversé la chambre de réaction, les gradients de champ magnétique sont éliminés, et en ce que les corps de fixation sont rassemblés dans une chambre collectrice, de préférence en-dessous de la chambre de réaction, pour un retraitement, spécialement pour éliminer les matières organiques déposées.

4. Procédé selon l'une quelconque des revendications précédentes, caractérisé en ce qu'un liquide de lavage traverse la chambre de réaction avant la traversée de la suspension ou de la solution.

5. Dispositif pour la mise en oeuvre du procédé selon l'une des revendications précédentes, comportant un compartiment de réaction (10) avec un orifice d'admission (20) et un orifice d'évacuation (22) pour faire tout d'abord passer le liquide dans lequel on veut effectuer la séparation et plus spécialement une suspension contenant des cellules-organelles ou protéines ou une solution nutritive sur un certain nombre de corps de fixation (36) magnétiques ou ferromagnétiques disposés à l'intérieur du compartiment de reaction (12) et un dispositif magnétique (14, 16, 38) destiné à produire un champ magnétique bloquant les corps de fixation (36) dans un volume réactionnel (34) à l'intérieur de compartiment de réaction (12), les corps de fixation étant pourvus d'une couche d'adsorption spécifique pour un ou plusieurs éléments à rechercher ou d'une couche pour la culture des cellules, caractérisé en ce qu'il est prévu un corps support (38) ferromagnétique, du type filigrane, remplissant le volume réactionnel (38) à la surface duquel sont retenus les corps de fixation (36) en raison des gradients de champ magnetique induits par le corps support (38).

6. Dispositif selon la revendication 5, caractérisé en ce que le champ magnétique ($H_0$) produit par le dispositif magnétique (14, 16, 38) passe transversalement par rapport à la direction d'écoulement de la suspension ou de la solution nutritive.

7. Dispositif selon la revendication 5 ou la revendication 6, caractérisé en ce que l'intensité du champ magnétique ($H_0$) se situe entre 0,05 et 0,5 Tesla et est de préférence d'environ 0,03 Tesla.

8. Dispositif selon l'une des revendications 5 à 7, caractérisé en ce que le corps de support est réalisé en fil métallique (38) et de preférence en fil d'acier spécial, dont le diamètre (d) se situe dans la zone des dimensions (diamètre e) des corps de fixation (36).

9. Dispositif selon la revendication 8, caractérisé en ce que le corps support présente la forme d'un grillage ou d'un tamis.

10. Dispositif selon la revendication 9, caractérisé en ce que le corps support présente la forme d'un fil courbé de façon arbitraire (38).

11. Dispositif selon l'une des revendications 8 à 10, caractérisé en ce que l'écartement moyen entre les morceaux de fil voisins est de 5 à 200 fois, et de préférence de 10 à 100 fois le diamètre (d) du fil métallique.

12. Dispositif selon l'une des revendications 4 à 10, caractérisé en ce que le compartiment de réaction (12) est disposé entre les pièces polaires (14, 16) d'un aimant.

13. Dispositif pour la mise en oeuvre du procédé selon l'une des revendications 1 à 4, comprenant un compartiment de réaction (110) avec un orifice d'admission (120) et un orifice d'évacuation (122) pour faire tout d'abord passer le liquide dans lequel on veut effectuer la séparation est plus spécialement une suspension contenant des cellules-organelles ou protéines ou une solution nutritive sur un certain nombre de corps de fixation (136) magnétiques ou ferromagnétiques disposés à l'intérieur du compartiment de réaction (112) et un aimant (142) pour la production d'un champ magnétique bloquant les corps de fixation (136) dans un volume réactionnel (134) à l'interieur du compartiment de réaction (112), les corps de fixation étant pourvus d'une couche d'adsorption spécifiques pour un ou plusieurs éléments à recherche ou d'une couche pour la culture des cellules, caractérisé en ce qu'un gradient de champ magnétique, diminuant depuis l'extérieur dans le cas d'un corps de fixation (136) ferromagnétique, est généré par le dispositif d'aimantation au moins sur la partie s'écoulant du volume reactionnel (134) où le liquide de séparation quitte le volume réactionnel (134), et en ce qu'il est prévu un dispositif d'agitation des corps de fixation (136) qui procède à la répartition des corps de fixation dans tout le volume réactionnel (134).

14. Dispositif selon la revendication 13, caractérisé en ce que le compartiment de réaction (112), de préférence cylindrique, est disposé dans l'alésage d'une bobine cylindrique (142).

15. Dispositif selon la revendication 13 ou la revendication 14, caractérisé par un barreau agitateur (146) rotatif, le cas échéant concentrique à l'axe du compartiment de réaction, qui déplace les corps de fixation (136) dans la direction opposée à la direction d'écoulement (C) du liquide de séparation.

16. Dispositif selon l'une des revendications 5 à 15, caractérisé par une ouverture d'entrée (26; 144), servant de préférence à l'aération du compartiment de réaction (12; 112) pour une aiguille creuse ou un tuyau souple pour l'introduction des corps de fixation (36; 130) dans le volume réactionnel (34; 134).

17. Dispositif selon l'une des revendications 5 à 16, caractérisé par une chambre-collecteur (40; 140) equipée de préférence d'une soupape de décharge (32; 132) pour les corps de fixation (36; 136) et située en-dessous du volume réactionnel (34; 134).

18. Dispositif selon l'un des revendications 5 à 17, caractérisé en ce que les dimensions (diamètre e) des corps de fixation (36; 136), de préférence sphériques, se situent entre 10 et 200 μm et de préférence entre 25 et 100 μm.

# FIG.1

# FIG. 2

# FIG.4

FIG.3